(19) Europäisches Patentamt
European Patent Office
Office européen des brevets

(11) EP 1 288 203 B1

(12) EUROPEAN PATENT SPECIFICATION

(45) Date of publication and mention of the grant of the patent:
**20.04.2005 Bulletin 2005/16**

(21) Application number: **02024174.1**

(22) Date of filing: **15.08.1996**

(51) Int Cl.7: **C07D 217/08**, C07D 491/04, C07D 495/04, A61K 31/473, A61K 31/4355, A61K 31/4365, A61P 39/06

# (54) Cyclic nitrones and pharmaceutical compositions containing them

Cyclische Nitrone und pharmazeutische Mittel die sie enthalten

Nitrones cycliques et compositions pharmaceutiques les contanant

(84) Designated Contracting States:
**AT BE CH DE DK ES FI FR GB GR IE IT LI LU MC NL PT SE**
Designated Extension States:
**SI**

(30) Priority: **11.09.1995 US 3551 P**

(43) Date of publication of application:
**05.03.2003 Bulletin 2003/10**

(62) Document number(s) of the earlier application(s) in accordance with Art. 76 EPC:
**96928900.8 / 0 863 878**

(73) Proprietor: **Aventis Pharmaceuticals Inc.**
**Bridgewater, NJ 08807-0800 (US)**

(72) Inventors:
- **Thomas, Craig E.**
  **West Chester, Ohio 45069 (US)**
- **Fevig, Thomas L.**
  **West Chester, Ohio 45069 (US)**
- **Bowen, Stephen M.**
  **Mohnton, PA 19540 (US)**
- **Farr, Robert A.**
  **Loveland, Ohio 45140 (US)**
- **Carr, Albert A.**
  **Cincinnati, Ohio 45237 (US)**
- **Janovick, David A.**
  **Beach Park, Illinois 60087 (US)**

(74) Representative: **Minoja, Fabrizio, Dr.**
**Bianchetti Bracco Minoja S.r.l.**
**Via Plinio, 63**
**20129 Milano (IT)**

(56) References cited:
**EP-A- 0 532 027**

- **THOMAS C E ET AL: "MULTIPLE MECHANISMS FOR INHIBITION OF LOW DENSITY LIPOPROTEIN OXIDATION BY NOVEL CYCLIC NITRONE SPINTRAPS" JOURNAL OF BIOLOGICAL CHEMISTRY, AMERICAN SOCIETY OF BIOLOGICAL CHEMISTS, BALTIMORE, MD, US, vol. 269, no. 45, 11 November 1994 (1994-11-11), pages 28055-28061, XP002018472 ISSN: 0021-9258**
- **THOMAS C E ET AL: "CHARACTERIZATION OF THE RADICAL TRAPPING ACTIVITY OF A NOVEL SERIES OF CYCLIC NITRONE SPIN TAPS" JOURNAL OF BIOLOGICAL CHEMISTRY, AMERICAN SOCIETY OF BIOLOGICAL CHEMISTS, BALTIMORE, MD, US, vol. 271, no. 6, 9 February 1996 (1996-02-09), pages 3097-3104, XP002018473 ISSN: 0021-9258**
- **THORNBER C W: "ISOTERISM AND MOLECULAR MODIFICATION IN DRUG DESIGN" CHEMICAL SOCIETY REVIEWS, CHEMICAL SOCIETY, LONDON, GB, vol. 8, no. 4, 1979, pages 563-580, XP000953019 ISSN: 0306-0012**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention is directed to cyclic nitrones, their use in the prevention of oxidative tissue damage by free radicals, their use in the treatment of a number of disease states in which radicals either damage or destroy tissues via oxidation, and pharmaceutical compositions containing these cyclic nitrones.

## BACKGROUND OF THE INVENTION

Molecules containing an unpaired electron are referred to as free radicals. Free radicals are extremely reactive. Partial reduction of oxygen by mammalian biological systems produces the free radicals, superoxide and hydroxyl. The two electron reduction product of oxygen, hydrogen peroxide, is also produced but contains no unpaired electrons. However, it is usually a precursor to the hydroxyl radical which is the most reactive of the three. The hydroxyl free radical will react with almost any biomolecule. Examples of such biomolecules include nucleic acids, lipids, and proteins. The hydroxyl radical will oxidize the biomolecule by either extracting a hydrogen atom from the biomolecule or by adding directly to the biomolecule itself. This oxidation by the hydroxyl free radical transforms the biomolecule into a radical which will readily react with molecular oxygen, thereby forming what is referred to as a peroxyl free radical. The resulting peroxyl radical will react with another biomolecule producing a free radical, which will also be transformed into another peroxyl radical as described above. The initial presence of the oxygen free radical initiates a chain reaction in which a number of biomolecules in the organism are oxidized. By oxidizing lipids, these free radicals can affect cell membranes, their permeability, ion channels, cell function, etc. By oxidizing proteins, they can alter enzymes, muscular function, nerves, etc. By oxidizing nucleic acids, they can affect DNA, RNA, and their expression products.

Recent research has indicated that excessive levels of these oxygen free radicals are associated with the tissue damage which occurs in a number of disease states such as stroke, myocardial infarction, senile dementia, shock, etc. Stroke and septic shock in particular are disease states in which radical-induced tissue damage is prevalent. Recent research has also shown that spin trapping agents may be utilized to terminate the reaction cascade described above, thereby preventing or minimizing any tissue damage. Oxygen free radicals and carbon centered radicals will react more readily with the spin trapping agent than with a biomolecule. The reaction with the spin trapping agent will result in the formation of a stable radical adduct and thus will terminate the chain reaction that is typically associated with oxygen radicals. Most tissue damage results from the chain reaction that is initiated by the oxygen radical rather than by the oxygen radical itself. The mechanism of action by which oxygen radicals cause tissue damage, as well as the use of spin trapping agents to prevent this damage, is described more fully by Floyd, FASEB Journal, Vol. 4, page 2588 (1990).

Nitrones 3,4-dihydro-3,3-dimethylisoquinoline N-oxide (**A**) and spiro [cyclohexane-1,3'] 3,4-dihydroisoquinoline N$^{-}$-oxide (**B**) (Figure 1) are cyclic analogs of the known radical scavenger PBN, which had previously been developed. Embedding the nitrone moiety in a cyclic system should give an essentially planar molecule in which good orbital overlap exists between the nitrone double bond and the aromatic ring. Molecular modelling studies indeed suggest that in the lowest energy conformation of **A**, the nitrone double bond is coplanar with the aromatic ring, whereas the corresponding relationship with PBN is ca. 30° offset from coplanarity. These predictions have been supported by X-ray crystallography. This increased degree of conjugation, relative to PBN, was expected to make the nitrone function in the cyclic analogs more accessible to radicals, and result in more stable product radicals. Experimentally, both **A** and **B** are more potent inhibitors of lipid oxidation, and better hydroxyl radical traps, than PBN. See U.S Patent No. 5,397,789, issued March 14, 1995, incorporated herein by reference.

**Figure 1**

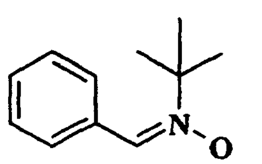

**PBN**

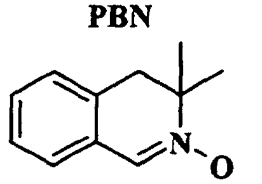

**A**

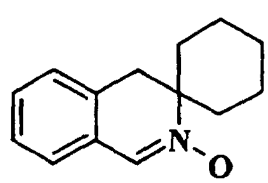

**B**

EP-A-532 027, that belongs to the same patent family of the above cited US patent, disclose, inter alia, cyclic nitrones of formula I wherein $R_1$ and $R_2$ each independently represents a $C_{1-3}$ alkyl or $R_1$ and $R_2$ together form a $C_{5-6}$ alkylene ring, the ring X represents phenyl optionally substituted with a substituent selected from $C_1$-$C_3$ alkyl, OH or $C_1$-$C_3$ alkoxy and $R_3$ represents hydrogen.

Similarly, Journal of Biological Chemistry, Vol 269, pages 28055-28061, 1994, describes the inhibition of low density lipoprotein oxidation by some of the nitrones described also in EP-A-532 027.

## SUMMARY OF THE INVENTION

The compounds disclosed herein are cyclic nitrones selected from:

2,2-dimethyl-1,2-dihydrobenzo[f]isoquinoline N-oxide;
3,3-dimethyl-3,4-dihydrobenzo[h]isoquinoline N-oxide;
5,5-dimethyl-4,5-dihydrothieno[2,3-c]pyridine N-oxide;
6,6-dimethyl-6,7-dihydrothieno[2,3-c]pyridine N-oxide;
5,5-dimethyl-4,5-dihydrofuro[2,3-c]pyridine N-oxide.

## DETAILED DESCRIPTION OF THE INVENTION

Two general methods are used to make the cyclic nitrones. The "formamide route" is used whenever possible.

The "isocyanate route" is used when the "formamide route" fails due to acid sensitivity of substrates or reaction intermediates. These routes will be described in detail for representative examples.

### The Formamide Route

The "formamide route" is illustrated below for the synthesis of the naphthalene compounds **9** and **10** (see Scheme **1**). Grignard addition to esters **1** and **2** provides tertiary alcohols **3** and **4** in good yield. Ritter reaction on these substrates with sodium cyanide affords the corresponding formamides **5** and **6.** Reaction of the formamides with oxalyl chloride, followed by cyclization with $FeCl_3$ and acid hydrolysis (to cleave the oxalate residue), provides the cyclic imines **7** and **8** (see Larsen, R.D.; Reamer, R.A.; Corley, E.G.; Davis, P.; Grabowski, E.J.J.; Reider, P.J.; Shinkai, I., J. Org. Chem. 1991, **56,** 6034). A single regioisomer is obtained in each case. Oxidation to the nitrones **9** and **10** proceeds more rapidly and efficiently when the imines are first reduced to the corresponding amines with sodium borohydride. It will be understood in the following schemes, examples and text that the sodium tungstate ($Na_2WO_4$) catalyst is the dihydrate form, i.e. $Na_2WO_42H_2O$.

## Scheme 1

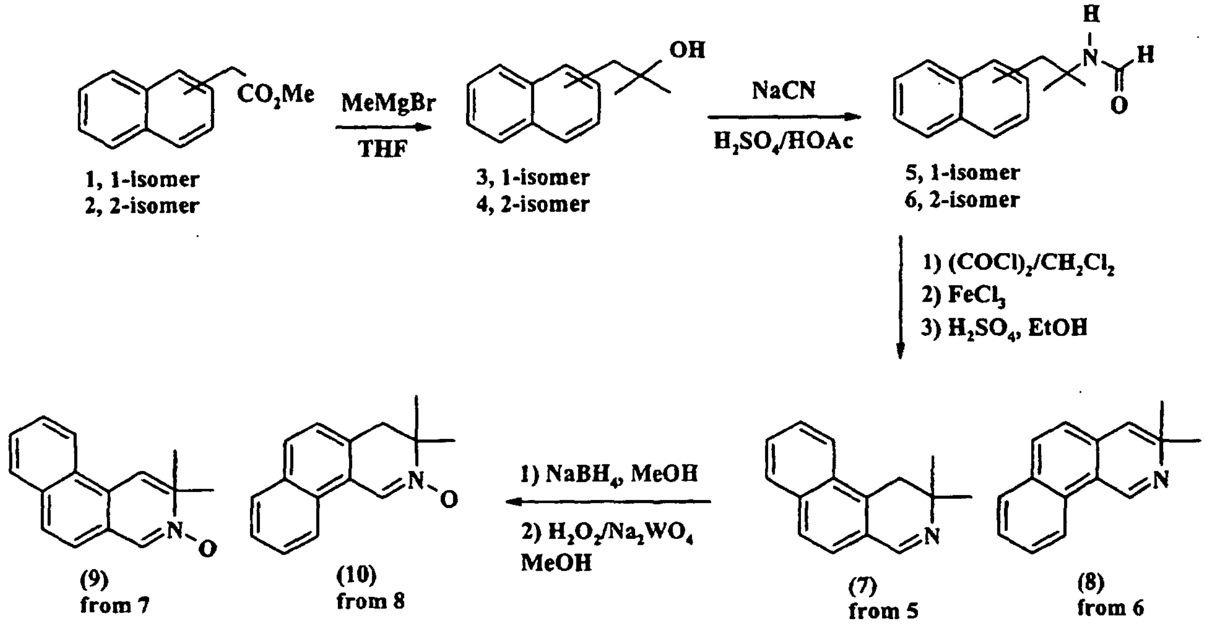


1, 1-isomer
2, 2-isomer

3, 1-isomer
4, 2-isomer

5, 1-isomer
6, 2-isomer

(9) from 7

(10) from 8

(7) from 5

(8) from 6

Compound **11** can also be prepared by this formamide route, or variations thereof.

## Figure 2

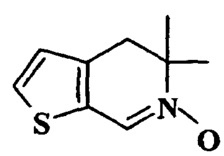

(11)

### The Isocyanate Route

The isocyanate route is shown in Scheme **2**. Alkylation of ethyl isobutyrate with chloromethyl thiophene, and subsequent hydrolysis of the ester (**14**), gives the Curtius rearrangement substrate **15**. The isocyanate **16** is formed smoothly, and is easily isolated after an aqueous work-up. Compound **16** cyclizes readily upon treatment with anhydrous $H_3PO_4$ in hot dichloroethane (DCE). Umezawa, B.; Hoshino, O.; Sawaki, S.; Mori, K. *Chem. Pharm. Bull.* **1980,** 28, 1003. The resulting lactam **17** is reduced to the corresponding amine with borane, whereupon standard oxidation gives **18.**

**Scheme 2**

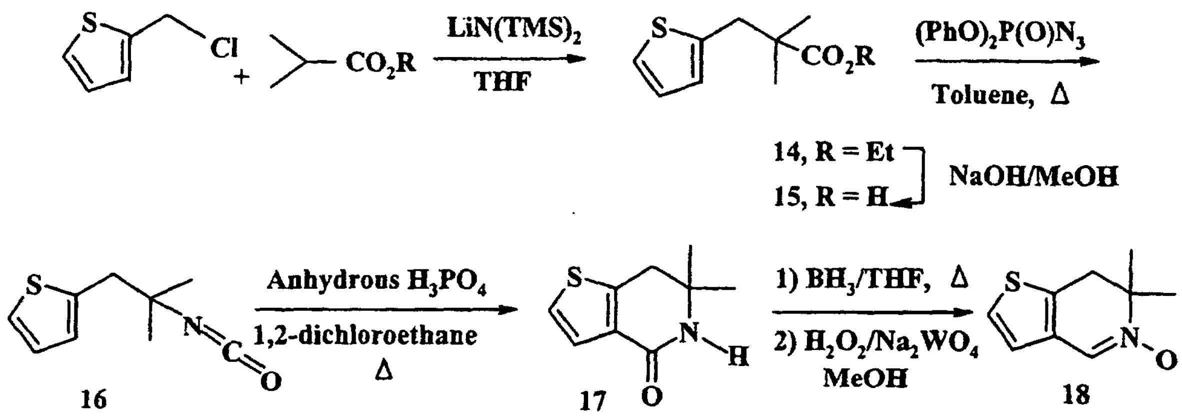


An analogous pathway is used to prepare the furan analog **23,** $BF_3$ etherate proving to be a better cyclization catalyst in this case.

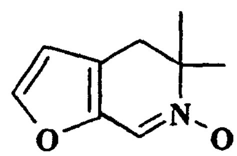

**23**

Compound **A** (see Figure **1**, supra) causes sedation when administered in high doses in rats. Indeed, from the onset, a primary goal has been to find a potent antioxidant which lacks this side effect observed with compound **A**. In this regard, the following observations were of special interest. The sedative effect peaks and declines rapidly, whereas the in vivo activity persists for a considerably longer period of time. The decline in sedation coincides with the appearance of a major metabolite of **A**, and this led to the speculation that the metabolite retains the antioxidant activity of the parent, but does not cause sedation.

**COMPOUND EVALUATION**

Oxidation of cellular macromolecules, including lipids and DNA, has been implicated in the etiology of numerous disease states. In the central nervous system (CNS), both stroke and neurotrauma have been proposed to initiate a sequelae of oxidative events which ultimately lead to neuronal cell death Kontos, H.A. (1989) *Chem.-Biol. Interactions* 72, 229-255. Brain neuronal membranes contain a high percentage of polyunsaturated fatty acids and an abundance of iron and ascorbic acid. Collectively, these properties yield neural tissue highly vulnerable to oxygen radical formation and lipid peroxidation. Iron and ascorbic acid can participate in the formation of radicals such as the hydroxyl radical (·OH) which are capable of initiating lipid peroxidation. Cellular responses to the ischemic or hypoxic environment which arise following the occlusion of an artery or following traumatic insult favor the generation of oxygen radicals. For example, aberrant mitochondrial electron transport due to lack of oxygen leads to a buildup in reducing equivalents which can partially reduce dioxygen to produce superoxide ($O_2{\cdot}^-$) and hydrogen peroxide ($H_2O_2$) upon reperfusion. Catecholamine accumulation, the conversion of xanthine dehydrogenase to its oxidase form, the release of arachidonic acid from phospholipids, and the attraction of neutrophils to ischemic tissue are other reported changes which all favor a highly oxidizing environment.

Animal models of global and focal ischemia have provided evidence for the production of oxygen radicals and the occurrence of lipid peroxidation. Increases in lipid- derived conjugated dienes and decreases in the protective antioxidant α-tocopherol (vitamin E) have been observed in rats subjected to brain ischemia-reperfusion. Hall, E.D. and Braughler, J.M. (1989) *J. Free Rad. Biol. Med.* 6, 303- 313. In agreement, brain tissue from animals rendered deficient in vitamin E was more susceptible to ischemia-induced damage while vitamin E supplementation had some protective effect. Yoshida, S., Busto, R., Watson, B.D., Santiso, M., and Ginsberg, M. (1985) *J. Neurochem.* 44,

1593-1601. Pentane evolved from lipid peroxidation has been found in the expired breath of gerbils subjected to global ischemia and reperfusion, which also supports the contention that neuronal lipid oxidation occurs under these conditions. Mickel, H. S., Vaishnav, S.Y.N., Kempski, O., von Lubitz, D., Weiss, J.F., and Feuerstein, G. (1987) *Stroke* 18, 426-430.

Oxidative events following CNS ischemia-reperfusion are not limited to lipids. In gerbils, global ischemia resulted in oxidation-induced protein carbonyl formation and in loss of activity of glutamine synthetase, an enzyme susceptible to oxidative inactivation. Oliver, C.N., Starke-Reed, P.E., Stadtman, E.R., Liu, G.J., Carney, J.M., and Floyd, R.A. (1990) *Proc. Natl. Acad. Sci USA* 87, 5144-5147.

Subjecting brain cortical slices to hypoxia and reoxygenation or injection of iron into CNS tissue leads to a loss of $Na^+$, $K^+$ - ATPase activity which may reflect direct protein oxidation and/or perturbation of the associated membrane bilayer. Taylor, M.D., Mellert, T.K., Parmentier, J.L., and Eddy, L.J. (1985) *Brain Res.* 346, 268-273; Anderson, D.K. and Means, E.D. (1983) *Neurochem. Pathol.* 1, 249-264.

While the evidence implicating oxygen radical formation as a cause of neuronal injury remains largely circumstantial, various antioxidant therapies have been tested for their ability to prevent or minimize loss of cell viability. As mentioned above, prior administration of vitamin E has been shown to provide partial protection. Limited success has been achieved with various forms of superoxide dismutase (SOD) and transgenic animals overexpressing SOD are more resistant to ischemia-induced injury. Kinouchi, H., Epstein, C.J., Mizui, T., Carlson, E., Chen, S.F., and Chan, P. H. (1991) *Proc. Natl. Acad. Sci.* USA 88, 11158-11162. Recently, investigators have reported that the nitrone spin trap alpha-phenyl-tert-butyl nitrone (PBN) can significantly ameliorate neuronal cell loss and neurologic deficits induced by stroke in a gerbil model. Phillis, J.W., and Clough-Helfman, C. (1990) *Med. Sci. Res.* 18, 403-404. Yue, T.-L., Gu, J. -L., Lysko, P.G., Cheng, H.-Y., Barone, F.C., and Feuerstein, G. (1992) *Brain Res.* 574, 193-197. Furthermore, PBN was shown by electron spin resonance (ESR) spectroscopy to trap lipid-derived radicals in cortical tissue of these animals.

Nitrone spin traps such as PBN have been utilized for a number of years to allow the trapping of short lived reactive radicals such as •OH. The resultant nitroxide is a more stable radical and can be detected by electron spin resonance spectroscopy. More recently, investigators have demonstrated that nitrones like PBN can inhibit the oxidation of lipids including low density lipoproteins and proteins such as glutamate synthetase. Thomas, C. E., Ku, G., and Kalyanaraman, B. (1994) *J. Lipid Res.* 35, 610-619; Thomas, C. E., Ohlweiler, D.F., and Kalyanaraman, B. (1994) *J. Biol. Chem.* **269,** 28055-28061 (use of antioxidants in the treatment of atherosclerosis); Carney, J-M., Starke-Reed, P. E., Oliver, C.N., Landrum, R.W., Cheng, M.S., Wu, J.F., and Floyd, R.A. (1991) *Proc. Natl. Acad. Sci. USA* 88, 3633-3636.

Another pathophysiologic situation wherein a role for oxygen radicals has been oft proposed is septic shock, which can be characterized as a systemic response to a serious infection. The resultant activation of inflammatory cells such as leukocytes is expected to result in the formation of $O_2 \cdot -$ and $H_2O_2$. Indeed, evidence of free radicals and free radical mediated tissue damage has been reported in animal models of endotoxic shock and in humans with septic shock. Takeda, K., Shimada, Y., Okada, T., Amono, M., Sakai, T., and Yoshiya, I. (1986) *Crit. Care Med*. 14, 719-723. Novelli, G.P., Angiolini, P., Livi, P., and Paternostro, E. (1989) *Resuscitation* 18, 195-205 Biasi, F., Chiarpotto, E., Lanfranco, G., Capra, A., Zummo, U., Chiappino, I., Scavazza, A., Albano, E., and Poli, G. (1994) *Free Rad. Biol. Med.* 17, 225-233. Interestingly, PBN has been demonstrated to reduce endotoxin associated mortality in rats. Hamburger, S.A., and McCay, P.B. (1989) *Circ. Shock* 29, 329-334. Thus, the use of spin traps like PBN may provide new therapeutics for the treatment of various disease states. See "Prospects for the Use of Antioxidant Therapies", *Drugs* **49(3)** 1995, 345-361. In addition, the ability of the nitrones to trap and stabilize radicals may provide a potential means to identify radicals which are generated *in vivo.* For these reasons, we have synthesized and evaluated a novel series of nitrone spin traps for *in vitro* radical trapping activity.

## MATERIALS AND METHODS

Cyclic nitrones were prepared as described above.

### Chemicals

2-Deoxy-D-ribose, $FeCl_2$, $FeCl_3$, disodium EDTA, 30% $H_2O_2$, ascorbic acid, thiobarbituric acid (TBA), 100% trichloroacetic acid (TCA) solution, butylated hydroxytoluene (BHT), NADPH, *p*-nitrosodimethylaniline (*p*-NDA), reduced glutathione (GSH), diethylenetriaminepentaacetic anhydride (DETAPAC), xanthine, xanthine oxidase (from buttermilk), N-methyl-D-glucamine, HEPES, 3-(4,5-dimethylthiazol-2yl)-2,5-diphenyl tetrazolium bromide (MTT), Cu,Zn-superoxide dismutase (SOD) and 1,1,3,3-tetraethoxypropane were purchased from Sigma Chemical Co. Soybean phosphatidylcholine was a product of Avanti Polar Lipids while PBN and β-cyclodextrins were 15 purchased from Aldrich. Cell culture supplies were obtained from Gibco or Sigma. All other chemicals were of the highest grade available.

Radical trapping *in vitro* by the cyclic nitrones was evaluated by: 1) examining the ability of the nitrones to inhibit oxidation of soybean phosphatidylcholine liposomes; 2) assessing •OH trapping using *p*-nitrosodimethylaniline or 2-deoxyribose and 3) ESR spin trapping for •OH and $O_2^{\cdot -}$.

1. Inhibition of lipid peroxidation

For determination of the ability to inhibit lipid peroxidation, liposomes were prepared from soybean phosphatidylcholine by ethanol injection. An aliquot of phosphatidylcholine was dried in a small glass vial under $N_2$. The lipid was resolubilized in ethanol at a volume of 10 ml per ml of liposomes. Typically, 8 ml volumes of liposomes were prepared per tube and then all preparations were combined to provide a homogenous mixture for the assay. The ethanol containing the lipid was taken up in a Hamilton syringe and injected into the appropriate volume of 50 mM NaCl/10 mM Tris, pH 7.0 at 37°C with mixing to achieve a final lipid concentration of 0.563 mM.

The liposomes were added to 25 ml beakers in a Dubnoff metabolic shaker at 37°C. To the liposomes were added the test compound (in ethanol or buffer), histidine-$FeCl_3$ 250:50 mM final), $FeCl_2$ (50 mM final, prepared in $N_2$ purged water) and sufficient buffer to achieve a final lipid concentration of 0.5 mM. Oxidations were initiated by the addition of $Fe^{2+}$ and carried out under an air atmosphere with shaking. One ml aliquots were removed at 0, 2, 4, 6, 8, 10, 12 and 15 min and added to 2 ml of 0.67% thiobarbituric acid : 10% trichloroacetic acid (2:1) in 0.25 N HCl, containing 0.05 ml of 2% BHT to terminate oxidation. Thomas, C.E., McLean, L.R., Parker, R.A., and Ohlweiler, D.F. (1992) *Lipids* 27, 543-550.

The samples were heated at 100°C for 20 min in 13 x 100 mm borosilicate glass tubes covered with marbles to prevent evaporation. After cooling, the tubes were centrifuged at 3,000 rpm for 10 min and the absorbance of the resultant supernatant read at 532 nm - 580 nm. Quantitation of thiobarbituric acid reactive substances (TBARS) was determined by comparison to a standard curve of malondialdehyde equivalents generated by acid catalyzed hydrolysis of 1,1,3,3-tetraethoxypropane. The $IC_{50}$ was determined using the 15 min time point with the computer program Graph-Pad INPLOT 4. This program uses a nonlinear regression with sigmoidal curve on a semilogarithmic scale. These results are tabulated in Table I.

2. Assessment as •OH Traps

A. Inhibition of the bleaching of p-NDA

Cyclic nitrones were evaluated for •OH trapping activity by a variety of tests. The primary assay was dependent upon the ability of the compounds to inhibit the •OH-dependent bleaching of *p*-NDA. Bors, W., Michel, C., and Saran, M. (1979) *J. Biochem.* 95, 621-627. The *p*-NDA was prepared at 1 mM in 50 mM NaCl, pH 7.0. The hydroxyl radical was generated using Fenton chemistry ($Fe^{2+}/H_2O_2$). $FeCl_2$ is dissolved in $N_2$ purged, double distilled $H_2O$ to a final concentration of 2.5 mM. $H_2O_2$ was prepared from a 30% stock solution (8.8 M) at 1.25 mM in the buffer. Test compounds were solubilized in buffer or ethanol at a concentration of 1 M or 5 M, depending upon solubility.

Assay mixtures in glass cuvettes contained solutions of 0.02 ml of $H_2O_2$, 0.02 ml of test compound, 0.10 ml of *p*-NDA and 50 mM NaCl, pH 7.0 to a final volume of 0.98 ml. The oxidation was initiated by the addition of 0.02 ml of $Fe^{2+}$ and the bleaching of *p*-NDA was monitored as the loss in absorbance at 440 nm for 100 sec. To generate concentration curves, serial dilutions of the test compounds were made such that a constant volume of 0.02 ml was added to the reaction mixture. Ethanol itself is an •OH trap, thus controls contained an equal volume of ethanol for any test compound requiring this vehicle. The $IC_{50}$ values for the nitrones were determined by GraphPad InPlot 4 and represent the amount of spin trap required to inhibit the bleaching of *p*-NDA by 50%.

B. Inhibition of 2-deoxyribose degradation

In this assay, hydroxyl radicals are also generated by Fentons's reaction. Their subsequent reaction with 2-deoxyribose causes this sugar molecule to break down to products reactive with TBA which can be measured spectrophotometrically. The $Fe^{3+}$ used in this assay is reduced to $Fe^{2+}$ by ascorbic acid and EDTA is used as an iron chelator to prevent site-specific damage directly to the deoxyribose molecule by the iron.

Stock Solutions

The buffer used in the incubation assay was a modified 30 mM Sorenson's buffer containing 40 mM NaCl, pH 7.4. It was prepared with 19% 30 mM $Na_2HPO_4$ and 81% 30 mM $NaH_2PO_4$, with NaCl added to yield a concentration of 40 mM.

Stock solutions were prepared as follows:

1) 100 mM 2-deoxyribose = 13.41 mg/ml buffer
2) 100 mM $H_2O_2$ = 50 µL 30% $H_2O_2$ solution + 4.4 ml buffer
3) 10 mM EDTA/10 mM $Fe^{3+}$ = 3.72 mg disodium EDTA+ 2.70 mg $FeCl_3.6H_2O$/ml buffer
4) 10 mM ascorbic acid = 1.761 mg/ml buffer
5) Radical scavengers to be assayed were prepared as stock solutions ranging from 5 to 100 mM in buffer, depending on their solubility. Organic solvents, such as methanol or ethanol, could not be used since even as small a volume as 5 ml of the solvent alone would substantially inhibit 2-deoxyribose degradation.
6) 0.378% TBA/ 15.2% TCA/ 0.014% BHT

   a) 16.7% TCA = 20 ml 100% TCA solution + 100 ml 0.125 N HCl
   b) 0.416% TBA = 0.500 g TBA + 120 ml 16.7% TCA solution (heated)
   c) 100 ml TBA/TCA solution + 10 ml 0.15% BHT in ethanol

7) 1.0 mM malondialdehyde (MDA) = 200 µl 4.4 mM MDA + 0.880 ml 10% TCA

   a) 4.4 mM MDA = 10 µl of 4.4 M 1,1,3,3-tetraethoxypropane + 9.99 ml 10% TCA

Standards

MDA standards were prepared as follows:

1) 0.0 nmoles/ml = 1.00 ml buffer
2) 10 nmoles/ml = 10 µl 1.0 mM MDA + 0.990 ml buffer
3) 25 nmoles/ml = 25 µl 1.0 mM MDA + 0.975 ml buffer
4) 50 nmoles/ml = 50 µl 1.0 mM MDA + 0.950 ml buffer

Incubations

Incubations were performed in 20 ml beakers in a shaking water bath set at 37°C and exposed to ambient air. The following constituents were added in the order listed: 1) buffer to make final volume 5.0 ml (4.71 ml for control); 2) 5 µl to 4.5 ml radical scavenger of interest (final concentration range of 5 µM to 4 mM); 3) 140 µl 100 mM 2-deoxyribose (2.8 mM); 4) 50 µl 100 mM $H_2O_2$ (1.0 mM); 5) 50 µl 10 mM EDTA/10 mM $Fe^{3+}$ (100 mM); and 6) 50 µl 10 mM ascorbic acid (100 mM).

At the 0 and 15 min time points after the addition of the ascorbic acid, 1.0 ml aliquots of incubation media were pipetted to tubes containing 2.0 ml TBA/TCA/BHT solution. Standards were also added to tubes containing 2.0 ml TBA/TCA/BHT. After vortexing, the covered samples and standards were heated in a heating block at 100°C for 20 min. The samples were cooled and centrifuged at 1500 x g for 10 min. Absorbances were read at $A_{532}$ - $A_{580}$. The radical scavenger concentration which inhibits TBARS formation by 50% ($IC_{50}$) was calculated using GraphPad INPLOT.

Determination of Rate Constant (k,) for Reaction of Hydroxyl Radicals With Radical Scavengers

When a hydroxyl radical scavenger is added to this reaction, a simple competition between the scavenger and the deoxyribose molecule takes place. From the method of Ching T., Halnen, G.R.M.M., and Bast, A. (1993) *Chem-Biol Interactions* 86, 119-127, the rate constant for the reaction of the scavenger with hydroxyl radical can be calculated with the equation:

$$1/A = 1/A^* (1 + k_s[S]/k_{DR}[D])$$

where

A = absorbance in presence of radical scavenger
[S] = radical scavenger concentration
A* = absorbance in absence of radical scavenger
ks = rate constant for reaction of scavenger with hydroxyl radical

$k_{DR}$ = rate constant for reaction of 2-deoxyribose with hydroxyl radical = 3.1 X $10^9$ $M^{-1}$ $set^{-1}$
[D] = 2-deoxyribose concentration = 2.8 mM

If 1/A is plotted against [S], then

$$slope = ks/k_{DR}[D]A^*$$

$$ks = slope \times k_{DR} \times [D] \times A^*$$

3. Protection of cerebellar granule cells against oxidative damage

The cyclic nitrones were tested for their ability to protect primary cultures of cerebellar granule cells against oxidative injury induced by treatment with $Fe^{2+}$. Cerebellar granule cell cultures were prepared from 8 day old rats as previously described. Levi, G., Aloisi, F., Ciotti, M.T., Thangnipon, W., Kingsbury, A., and Balazs, R. (1989) In: *A Dissection and Tissue Culture Manual of the Nervous System* (Shahar, A., de Vellis, J., Vernadakis, A., and Haber, B., eds.) Alan R. Liss, Inc. New York, NY, pp. 211-214. Briefly, 8-10 cerebella were removed and placed in a Crebs-Ringer bicarbonate medium supplemented with BSA and $MgSO_4$. Cerebella were finely chopped and digested with a trypsin/Krebs-Ringer solution. Cells were then dispersed by trituration in Krebs-Ringer containing DNase, $MgSO_4$ and trypsin inhibitor followed by plating at a density of $1x10^6$ cells/well in poly-1-lysine coated plate in MEM/10% fetal bovine serum/KCl/glutamine/gentamicin. Media was replaced at 24 hours and cytosine arabinoside added. Experiments were conducted with cells 8-10 days *in vitro.*

For oxidation studies the media was removed and replaced with $Na^+$ free Locke's solution (154.6 mM N-methyl-D-glucamine, 5.6 mM KCl, 2.3 mM $CaCl_2$, 1 mM $MgCl_2$, 3.6 mM $NaHCO_3$, and 5 mM HEPES, pH 7.3) with the omission of glucose. The nitrones were added in either Locke's solution or in 20% β-cyclodextrins and allowed to incorporate into the cells for 30 min. At this time 20 μl of a 5 mM stock solution of ferrous chloride was added for a final concentration of 100 μM. After 45 min the media was removed and added to 1.5 ml of TBA/TCA (2:1) with 25 μl of 2% BHT and TBARS determined as described above. The absorbance of the control cells (no iron) was subtracted from the iron treated cells and this value taken as the denominator to determine the concentration of nitrone required to inhibit oxidation by 50%.

Fresh MEM media was added to the cells along with 100 μl of MTT. After 4 hrs, 1 ml of cold isopropanol/0.04 N HCl was added, the cells scraped, mixed well and transferred to 13 x 100 mm glass test tubes. The absorbance resulting from mitochondrial reduction of the MTT (570 nm minus 630 nm) was measured as an assessment of viability. The % cell death was determined by comparison to the absorbance of cells to which no iron was added. The difference between the iron-treated and control cells was taken as 100% and the concentration of nitrone to prevent the loss of MTT reduction capability (expressed in Table II as viability) by 50% was used as the $IC_{50}$.

The cyclic nitrones that were prepared are listed in **Table I** along with their $IC_{50}$ values for inhibition of lipid oxidation and hydroxyl radical trapping in vitro. MDL 101,002 is included for comparison.

**TABLE I**

| COMPOUND | Compound # | LIPID OX ($IC_{50}$) | OH RADICAL TRAPPING |
|---|---|---|---|
| 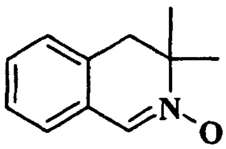 | A | 1.67 mM | 2.83 mM |
| 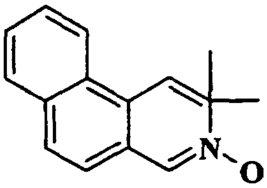 | 9 | 0.1 mM | not measured<br>low solubility |
| 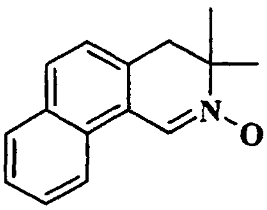 | 10 | 0.069 mM | not measured |
| 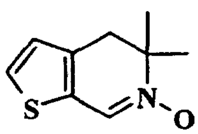 | 11 | 1.72 mM | 4.34 mM |
| 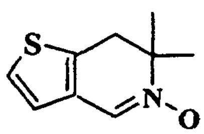 | 18 | 1.59 mM | 14.4 mM |
| 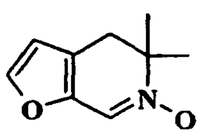 | 23 | 1.07 mM | no effect at 20 mM |

## TABLE II

**$IC_{50}$ Values for Inhibition of $Fe^{2+}$-Induced Damage to Cerebellar Granule Cells**

| Compound | *TBARS $IC_{50}$ (μM)* | *Viability* |
|---|---|---|
| PBN | 2600 | 2600 |
| A (R = H) | 307 | 292 |
| 10 (naphthyl) | 104 | 103 |
| 18 (sulfophenyl) | 2600 | 880 |

The compounds of the present invention may be administered by a variety of routes. They are effective if administered orally. The compounds may also be administered parenterally (i.e. subcutaneously, intravenously, intramuscularly, intraperitoneally, or intrathecally).

Pharmaceutical compositions can be manufactured utilizing techniques known in the art. Typically a protective amount of the compound will be admixed with a pharmaceutically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations.

In another embodiment, the compounds of the invention can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane, is described in U.S. Pat. No.3,921,636. At least two types of release are possible in these systems. Release by diffusion occurs when the matrix is non-porous. The pharmaceutically effective compound dissolves in and diffuses through the matrix itself. Release by microporous

flow occurs when the pharmaceutically effective compound is transported through a liquid phase in the pores of the matrix.

The compounds of the invention may be administered in the amount of from 0.01 mg/kg to 500 mg/kg, depending on a variety of factors such as weight, age, sex, condition being treated, etc.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention applies.

As used in this application:

a) the term "patient" refers to warm blooded animals such as, for example, guinea pigs, mice, rats, gerbils, cats, rabbits, dogs, monkeys, chimpanzees, and humans;
b) the term "treat" refers to the ability of the compounds to either relieve, alleviate, or slow the progression of the patient's disease or any tissue damage associated with the disease;
c) the term "neurodegeneration" refers to a progressive death and disappearance of a population of nerve cells occurring in a manner characteristic of a particular disease state and leading to brain or other neuronal damage;
d) the term 'shock" is used to refer to circulatory shock, septic shock, toxic shock, or any other condition in which oxygen derived radicals lead to inadequate perfusion of vital organs by the circulatory system;
e) the term "oxygen free radical" should be construed as referring to carbon centered radicals, oxygen radicals, or any biomolecule containing an unpaired electron in any discussion of tissue damage.

The compounds of the invention may also be admixed with any inert carrier and utilized in laboratory assays in order to determine the concentration of the compounds within the serum, urine, etc., of the patient as is known in the art. The compounds may also be used as a research tool by forming adducts with molecular oxygen.

## EXPERIMENTAL

### General Methods

Except where noted otherwise, reagents and starting materials are obtained from common commercial sources and used as received. Tetrahydrofuran (THF) is distilled from sodium-benzophenone ketyl immediately prior to use. Other reaction solvents, and all chromatographic, recrystallization and work-up solvents are spectroscopic grade and used as received. Reactions reported as being run "under $N_2$" are carried out under an atmosphere of dry $N_2$ in oven-dried flasks.

Thin layer chromatography (TLC) is performed on glass-backed, silica gel 60F-254 plates (EM) coated to a thickness of 0.25 mm. The plates are eluted with solvent systems (v/v) as described, and visualized by one or more of the following methods: W light, $I_2$ vapor, or staining with phosphomolybdic acid, $Ce(SO_4)_2$, $KMnO_4$, or $FeCl_3$ solutions, followed by heating (heat gun). "Thin-Layer Chromatography, a Laboratory Handbook", Egon Stahl, Ed.Springer-Verlag Berlin Heildelberg-New York, 1969. Gas chromatography (GC) is performed on a Hewlett Packard 5890 Series II gas chromatograph equipped with a Hewlett Packard 3392A integrator. Separations are carried out on a 15 m x 0.32 mm ID fused silica capillary column (DB-5, 0.25 mm film) from J & W Scientific.

"Concentrated *in vacuo*" and similar phrases indicate rotary evaporation on a Buchi apparatus at ca. 50°C and 15-20 Torr (water aspirator), unless stated otherwise. Flash chromatography (FC) is carried out using EM Science silica gel 60 (40 - 63 um) according to the literature procedure. Still, W. C.; Kahn, M.; Mitra, A. J. *Org. Chem.* **1978,** 43, 2923.

Melting points are determined on a Thomas Hoover Uni-melt capillary melting point apparatus. Melting points and boiling points are reported uncorrected.

IR spectra are recorded on a Mattson Galaxy Series 5020 infrared spectrophotometer with samples prepared as indicated, and are reported in wavenumbers ($cm^{-1}$). $^1$H NMR spectra are recorded on a Varian Gemini instrument (300 MHz) with chemical shifts (δ) reported in ppm relative to tetramethylsilane (0.00 ppm) or chloroform (7.26 ppm), unless stated otherwise. Signals are designated as s (singlet), d (doublet), t (triplet), q (quartet), p (pentet 1, m (multiplet), br (broad), etc. Coupling constants (J) are reported in Hz. First order analyses of spectra are attempted when possible; consequently, chemical shifts and coupling constants for multiplets may only be approximate. $^{13}$C NMR spectra are recorded on the Varian Gemini instrument (75 MHz) with chemical shifts (δ) reported in ppm relative to chloroform-d (77.00 ppm), unless stated otherwise. Mass spectra (MS) are obtained on a Finnigan MAT Model TSQ 700 Mass Spectrometer System using electron impact or chemical ionization with the molecular ion designated as M given in parentheses.

**General Procedure for the Reaction of MeMgBr with Esters**

**(Procedure A).**

A solution of MeMgBr (2.5 equiv of a 3 M $Et_20$ solution) THF (equal volume) is placed under $N_2$. The solution is cooled to -78°C, and the substrate (1 equiv) is added, either neat or as a solution in THF. The cooling bath and is removed, and the reaction mixture is allowed to warm to room temperature (rt). Excess MeMgBr is quenched by adding saturated $NH_4Cl$ solution, and the resulting mixture is poured into dil. HCl and extracted with EtOAc (2x). The organic phase is washed with saturated NaCl solution (brine), dried ($MgSO_4$ or $Na_2SO_4$), filtered and evaporated. The residue is purified as indicated.

**General Procedure for the Ritter Reaction (Procedure B).**

Powdered NaCN (1.5 to 2.5 equiv) is placed in a dry flask under $N_2$, and cooled in an ice bath. Acetic acid (HOAc) is added, and the mixture is stirred vigorously while a previously prepared mixture of conc. $H_2SO_4$ in an equal volume of HOAc is added through a dropping funnel (Caution! HCN generated). The substrate (1 equiv) is then added, either neat or in a minimum volume of HOAc, the cooling bath is removed, and the mixture is stirred at rt until TLC analysis indicates that the reaction is complete. Excess HCN is then evaporated under a stream of $N_2$ for 1-2 h. The residue is added slowly to a saturated solution of $NaHCO_3$ (vigorous gas evolution), and the mixture is extracted thoroughly with EtOAc. The organic phase is washed with brine, and dried ($Na_2SO_4$), filtered, and concentrated. The residue is purified as indicated.

**General Procedure for Cyclization of Formamides (Procedure C).**

The formamide (1 equiv) is dissolved in $CH_2Cl_2$ under $N_2$, and cooled in an ice bath. Neat oxalyl chloride (1.1 equiv) is added via syringe, the cooling bath is removed, and the mixture is stirred at rt for 1-2 h. The mixture is then cooled again to 0°C, and solid $FeCl_3$ (1.2 equiv) is added in one portion. The cooling bath is removed, and the mixture is stirred at rt overnight. The resulting reaction mixture is poured into 0.5 M HCl solution and extracted with EtOAc (2x). The organic phase is washed with brine, dried ($Na_2SO_4$), filtered, and evaporated. The residue is taken up in EtOH, treated with a catalytic amount of conc. $H_2SO_4$, and heated at reflux until TLC analysis indicates complete reaction (ca. 3 h). The mixture is then cooled, poured into saturated $NaHCO_3$ solution and extracted with EtOAc (3x). The organic phase is washed with brine, dried ($Na_2SO_4$), filtered, and evaporated. The residue is purified as indicated.

**General Procedure for Reduction of Imines (Procedure D).**

The imine (1 equiv) is dissolved in MeOH under $N_2$. Solid $NaBH_4$ (1.5 equiv) is added to the solution in small portions (gas and heat evolution). The resulting mixture is stirred at rt for 1-2 h, then added cautiously to 1 M HCl solution. The aqueous phase is washed with EtOAc (discarded), and made basic by adding KOH pellets. The liberated free amine is extracted into EtOAc (3x). The organic phase is washed with brine, dried ($Na_2SO_4$), filtered, and evaporated, to give the amine which is used as such.

**General Procedure for Oxidation of Amines to Nitrones (Procedure E).**

The amine (1 equiv) is dissolved in MeOH and treated sequentially with $Na_2WO_4$ (0.1 equiv) and 30% $H_2O_2$ (3 equiv). The resulting mixture is stirred at rt until TLC analysis indicates complete reaction (ca. 4 h). The reaction mixture is poured into brine containing $Na_2S_2O_3$ (to destroy excess peroxide) and extracted several times with EtOAc (until aqueous phase shows little or no product by TLC). The organic phase is dried ($Na_2SO_4$), filtered, and evaporated. The crude product is purified as indicated.

**General Procedure for Alkylation of Ethyl Isobutyrate (Procedure F).**

To a solution of lithium (bis)trimethylsilylamide ($LiN(TMS)_2$, 1 M solution in THF, 1.5 equiv) in THF cooled at -78°C under $N_2$ is added ethyl isobutyrate (1 equiv). Stirring is continued at -78°C for 1 h, then 1,3-dimethyl-3,4,5,6-tetrahydro-2(1H)-pyrimidinone (DMPU, 2% by volume) is added, followed by the electrophile. The cooling bath is removed, and the reaction mixture is stirred overnight, The reaction mixture is poured into cold 1 M HCl and extracted with EtOAc (3x). The organic phase is washed with water and brine, then dried ($MgSO_4$), filtered and evaporated. The residue is purified by FC ($CH_2Cl_2$).

**General Procedure for Hydrolysis of Ethyl Esters (Procedure G).**

The ester (1 equiv) is added to a solution of KOH (2.5 equiv) in 10% aqueous MeOH, and the resulting mixture is heated at reflux until TLC shows the absence of starting material. The mixture is cooled, and most of the MeOH evaporated. The residue is diluted with water and extracted with $Et_2O$ (2x, discarded). The aqueous phase is acidified by adding dilute HCl, and extracted with EtOAc (3x) - The organic phase is washed with brine, dried ($MgSO_4$), filtered, and evaporated. The crude product is used without further purification.

**General Procedure for the Curtius Rearrangement (Procedure H).**

To a solution of the carboxylic acid (1 equiv) in toluene at 0°C and under $N_2$ are added $Et_3N$ (0.95 equiv) and diphenyl phosphorylazide (0.95 equiv). The mixture is stirred at 0°C for 30 min, then heated at reflux for 3 h. The mixture is cooled, washed with cold $NaHCO_3$ solution (2x) and brine (2x), then dried ($MgSO_4$), filtered, and evaporated. The crude product is used without purification.

**General Procedure for Reduction of Lactams (Procedure I).**

The lactam (1 equiv) is carefully (gas evolution) added to $BH_3$.THF solution (1 M in THF, 2.5 equiv) under $N_2$. After gas evolution subsides, the mixture is heated at reflux overnight. The reaction mixture is cooled, treated cautiously with MeOH (ca. 50% by volume) and 1 M NaOH solution, and heated at reflux for 7 h. The resulting mixture is cooled and extracted with EtOAc (2x). The organic phase is extracted with 1 M HCl (2x), and the aqueous phase is neutralized by adding $NaHCO_3$. The product is extracted into EtOAc (2x), and the organic phase is washed with brine, dried ($MgSO_4$), filtered, and evaporated. The amines are used without further purification.

The following examples further illustrate the reaction sequences described above. However, they should not be construed as limiting the invention in any manner.

**<u>EXAMPLE 1</u>**

**2,2-Dimethyl-1,2-dihydrobenzo[f]isoquinoline N-oxide (9)**

**2-Methyl-1-naphthalen-1-yl-propan-2-ol (3)**

Ester **1** (see Acton, N.; Berliner, E. J. Am. Chem. Soc. 1984, 86, 3312) (20.0 g, 100 mmol) is treated with MeMgBr according to general procedure A. The product 3 is obtained as a white solid, mp 47-48°C, after evaporation of solvent. It requires no further purification. Yield: 19.4 25 g (97%). $^1$H NMR ($CDCl_3$) 8.16 (d, 1, J = 7.5), 7.85-7.75 (m, 2), 7.50-7.35 (m 4), 3.27 (s, 2), 1.27 (s, 6); $^{13}$C NMR ($CDCl_3$) 134.18, 133.96, 133.06, 129.01, 128.60, 127.29, 125.70, 125.42, 125.15, 125.04, 71.64, 45.08, MS 29.69; (MW= 200.3, CI/$CH_4$, eE=70 eV) *m*/*z* 200 (M+), 185, 184, 183, 171, 167, 155, 143, 142 (base peak), 115,89.

**N-(1,1-Dimethyl-2-naphthalen-1-yl-ethyl)-formamide (5)**

Alcohol **3** (3.00 g, 15.0 mmol) is subjected to the Ritter reaction according to general procedure **B**. The product **5** is obtained as a tan solid, mp 79-80°C, after FC (1:1, hexane/EtOAc). Yield: 2.76 g, 81%. The following $^1$H NMR spectrum is for a ca. 70:30 mixture of amide rotamers. Signals for the major rotamer are designated A, and those for the minor rotamer are designated B. $^1$H NMR ($CDCl_3$) 8.18 (d, 0.7, J = 8.5, A), 8.05-8.00 (m, 1.3), 7.85-7.75 (m, 2), 7.55-7.33 (m, 4), 5.90 (br d, 0.3, J=9.0, B), 5.25 (br s, 0.7, A), 3.56 (s, 1.4, A), 3.29 (s, 0.6, B), 1.40 (s, 4.2, A), 1.39 (s, 1.8, B); MS (MW=227.3, EI, eE=70 eV) *m*/*z* 227 (M+), 209, 183, 182, 167, 165, 141, 139, 128, 115, 89, 86 (base peak), 76, 63, 58, 42.

**2,2-Dimethyl-1,2-dihydrobenzo[f]isoquinoline (7)**

Formamide **5** (2.27 g, 10.0 mmol) is cyclized according to general procedure **C.** The imine **7** (1.61 g, 77%) is obtained as a dark brown solid after flash chromatography (FC) (19:1, $CH_2Cl_2$/MeOH). $^1$H NMR ($CDCl_3$) 8.31 (s,1), 8.08 (d, 1, J=7.8), 7.90-7.75 (m, 2), 7.60-7.50 (m, 2), 7.42 (d, 1, J=8.1), 3.12 (s, 2), 1.35 (s, 6); $^{13}$C NMR ($CDCl_3$) 157.76, 134.78, 128.57, 126.85, 126.34, 124.32, 124.23, 123.79, 123.41, 54.75, 33.69, 28.49; MS (MW=209.3, EI, e=70 eV) *m*/*z* 209 (M+, base peak), 194, 181, 167, 152, 139, 115, 97, 82, 75, 63, 41.

**2,2-Dimethyl-1,2,3,4-tetrahydrobenzo[f]isoquinoline**

Reduction of imine **7** (1.61 g, 7.7 mmol) according to general procedure **D** delivers the amine (1.61 g, 100%) as a dark liquid which is not characterized, but used directly in the next reaction.

**2,2-Dimethyl-1,2-dihydrobenzo[f]isoquinoline N-oxide (9)**

The crude amine from the previous reaction (1.481 g, 7.024 mmol) is oxidized according to general procedure **E** to give a tan solid, mp 155-157°C, after recrystallization from 1:9 $CH_2Cl_2$/hexane. Yield: 0.866 g (55%). $^1$H NMR ($CDCl_3$) 7.96 (d, 1, J=8.3), 7.85-7.75 (m, 2), 7.81 (s, 1), 7.55-7.50 (m, 2), 7.22 (d, 1, J=8.5), 3.45 (s, 2), 1.54 (s, 6); $^{13}$C NMR ($CDCl_3$) 133.76, 132.95, 130.75, 128.89, 127.80, 127.09, 126.45, 125.72, 125.47, 123.25, 122.55, 66.89, 38.32, 25.42; MS (EI, eE=70 eV) *m/z* 225 (M+, base peak), 210, 194, 193, 165, 139, 115, 89, 76, 63, 41;

| | | | |
|---|---|---|---|
| Anal. Calcd for $C_{15}H_{15}NO$ (MW=225.3) | C, 79.97 | H, 6.71 | N, 6.22. |
| Found | C, 78.79 | H, 6.66 | N, 6.22. |

**EXAMPLE 2**

**3,3-Dimethyl-3,4-dihydrobenzo[h]isoquinoline N-oxide (10)**

**2-Methyl-1-naphthalen-2-yl-propan-2-ol (4)**

Ester **2** (see Acton, N.; Berliner, E. J. Am. Chem. Soc. 1984, 86, 3312) (7.87 g, 39.3 mmol) is treated with MeMgBr according to general procedure **A**. The product **4** is obtained as a white solid, mp 79-80°C, after evaporation of solvent. It requires no further purification. Yield: 5.88 g (75%). $^1$H NMR ($CDCl_3$) 7.80-7.75 (m, 3), 7.66 (s, 1), 7.45-7.35 (m, 3), 2.92 (s, 2), 1.26 (s, 6); $^{13}$C NMR ($CDCl_3$) 135.37, 133.33, 132.19, 129.06, 128.80, 127.57, 125.96, 125.46, 70.95, 49.80, 29.25; MS (MW=200.3, EI, e=70 eV) *m/z* 200 (M+), 185, 167, 165, 143, 142, 141 (base peak), 128, 115, 89, 63, 59, 57, 43, 31.

**N-(1,1-Dimethyl-2-naphthalen-2-yl-ethyl)-formamide (6)**

Alcohol **4** (3.00 g, 15.0 mmol) is treated with NaCN according to general procedure **B.** The product **6** is obtained as a yellow solid (3.31 g, 97%), mp 59-63°C, after FC ($CH_2Cl_2$). The following $^1$H NMR spectrum is for a ca. 67:33 mixture of amide rotamers. Signals for the major rotamer are designated A, and those for the minor rotamer are designated B. $^1$H NMR ($CDCl_3$) 8.10-8.05 (m, 1), 7.80-7.75 (m, 3), 7.60 (m, 1), 7.50-7.45 (m, 2), 7.30-7.25 (m, 1), 5.95 (br s, 0.33, B), 5.22 (br s, 0.67, A), 3.20 (s, 1.3, A), 2.92 35 (s, 0.7, B), 1.40-1.30 (m, 6); MS (MW=227.3, EI, eE=70 eV). *m/z* 227 (M+), 209, 183, 182, 152, 141, 139, 115, 89, 86 (base peak), 63, 58, 42, 32.

**3,3-Dimethyl-3,4-dihydrobenzo[h]isoquinoline (8)**

Cyclization of formamide **6** (2.27 g, 10.0 mmol) according to general procedure **C** furnishes the imine **8** (1.55 g, 74%) as a tan solid which is used without purification. $^1$H NMR ($CDCl_3$) 9.08 (s, 1), 8.31 (d 1, J=8.4), 7.90-7.85 (m, 2), 7.60-7.55 (m, 1), 7.50 (m, 1), 7.29 (s, 1), 2.88 (s, 2), 1.30 (s, 6); $^{13}$C NMR ($CDCl_3$) 153.38, 134.69, 132.61, 131.07, 129.12, 128.50, 127.04, 126.44, 125.18, 121.34, 121.02, 53.97, 38.59, 27.71; MS (MW=209.3, EI, eE=70 eV) *m/z* 209 (M+, base peak), 194, 180, 167, 152, 139, 115, 97, 82, 76, 63,51,41.

**3,3-Dimethyl-1,2,3,4-tetrahydrobenzo[h]isoquinoline**

Reduction of imine **8** (1.47 g, 7.03 mmol) according to general procedure **D** delivers the amine (1.47 g, 99%) as a dark liquid which is not characterized, but used directly in the next reaction.

**3,3-Dimethyl-3,4-dihydrobenzo[h]isoquinoline N-oxide (10)**

The crude amine from the previous reaction (1.47 g, 6.97 mmol) is oxidized according to general procedure **E** to afford a yellow solid, mp 157-159°C, after recrystallization from 1:9. $CH_2Cl_2$/hexane. Yield: 0.950 g (61%). $^1$H NMR ($CDCl_3$) 8.51 (s, 1), 7.97 (d, 1, J=8.5), 7.85-7.75 (m, 2), 7.55-7.50 (m, 2), 7.31 (d, 1, J=8.3), 3.21 (s, 2), 1.50 (s, 6); $^{13}$C NMR ($CDCl_3$) 132.64, 129.51, 129.27, 128.75, 128.15, 127.58, 125.90, 125.63, 123.06, 121.28, 66.15, 42.34,

24.45; MS ($CI/CH_4$, eE=70 eV) *m/z* 226 [(M+H)+, base peak], 210, 193, 167, 152;

| | | | |
|---|---|---|---|
| Anal. Calcd for $C_{15}H_{15}NO$ (MW=225.3) | C, 79.97 | H, 6.71 | N, 6.22. |
| Found | C, 79.58 | H, 6.72 | N, 6.02. |

## REFERENCE EXAMPLE 3

**5,5-Dimethyl-4,5-dihydrothieno[2,3-c]pyridine N-oxide (11)**

**2-Methyl-1-thiophen-3-yl-propan-2-ol (12)**

The ethyl ester of thiophene-3-acetic acid (15.0 g, 88.2 mmol) is treated with MeMgBr according to general procedure **A**. The product **12** is obtained as a colorless liquid (16.1 g, 88%) which requires no further purification. $^1H$ NMR ($CDCl_3$) 7.25-7.20 (m, 1), 7.00-6.95 (m, 2), 2.77 (s, 2), 1.21 (s, 6); $^{13}C$ NMR ($CDCl_3$) 138.39, 130.18, 125.39, 123.12, 70.77, 44.42, 29.37; MS (MW=156.2, EI, eE=70 eV) *m/z* 156 (M+), 141, 139, 100, 98 (base peak), 97, 85, 69, 59, 43, 32.

**5,5-Dimethyl-4,5-dihydrothieno[2,3-c]pyridine (13)**

Treatment of the alcohol **12** from the previous reaction (15.9 g, 102 mmol) with NaCN according to general procedure **B** gives the cyclized imine directly as a dark liquid after FC (3:1 hexane/EtOAc). Yield: 4.15 g (25%). $^1H$ NMR ($CDCl_3$) 8.17 (s, 1), 7.36 (d, 1, J=4.8), 6.88 (d, 1, J=4.8), 2.75 (s, 2), 1.28 (s, 6); $^{13}C$ NMR ($CDCl_3$) 153.96, 141.02, 131.45, 125.79, 124.39, 56.24, 35.65, 28.03; MS (MW=165.3, EI, eE=70 eV) *m/z* 165 (M+, base peak), 150, 138, 124, 123, 97, 86, 77, 69, 58, 45.

**5,5-Dimethyl-4,5,6,7-tetrahydrothieno[2,3-c]pyridine**

Reduction of the imine **13** from the previous reaction (1.00 g, 6.1 mmol) according to general procedure **D** provides the corresponding amine (1.00 g, 100%) as a dark liquid (MW=167.3) which is used without further purification. $^1H$ NMR ($CDCl_3$) 7.13 (d, 1, J=5.1), 6.75 (d, 1, J=5.1), 4.07 (s, 2), 2.56 (s, 2), 2.37 (s, 1), 1.23 (s, 6).

**5,5-Dimethyl-4,5-dihydrothieno[2,3-c]pyridine N-oxide (11)**

The crude amine from the previous reaction (1.00 g, 5.99 mmol) is oxidized according to general procedure **E** to afford a tan solid (300 mg, 28%, mp 136-138°C) after FC (19:1 $CH_2Cl_2$/MeOH). $^1H$ NMR ($CDCl_3$) 7.73 (s, 1), 7.33 (d, 1, J=4.9), 6.93 (d, 1, J=4.9), 3.04 (s, 2), 1.48 (s, 6); $^{13}C$ NMR ($CDCl_3$) 132.25, 128.30, 127.52, 127.14, 126.64, 68.03, 38.24, 25.25; MS (EI, eE=70 eV) *m/z* 181 (M+, base peak), 166, 149, 138, 134, 110, 96, 91, 77, 65, 51, 45;

| | | | | |
|---|---|---|---|---|
| Anal. Calcd for $C_9H_{11}NOS$ (MW=181.3) | C, 59.64 | H, 6.12 | N, 7.73 | S, 17.69. |
| Found | C, 59.57 | H, 6.10 | N, 7.86 | S, 17.56. |

## REFERENCE EXAMPLE 4

**6,6-Dimethyl-6,7-dihydrothieno[3,2-c]pyridine N-oxide (18)**

**3-Thiophen-2-yl-2,2-dimethylpropionic acid ethyl ester (14)**

Ethyl isobutyrate (10.9 ml, 81.7 mmol) is alkylated with 2-(chloromethyl)thiophene according to general procedure **F** to give the product **14** as a yellow liquid (16.4 95%). $^1H$ NMR ($CDCl_3$) 7.15-7.10 (m, 1), 6.95-6.90 (m, 1), 6.77 (d, 1, J=3.6), 4.15 (t, 2, 5=7.0), 3.07 (s, 2), 1.26 (t, 3, J=7.0), 1.21 (s, 6); $^{13}C$ NMR ($CDCl_3$) 176.96, 139.80, 126.74, 126.42, 123.94, 60.59, 43.65, 40.30, 25.07, 14.22; MS (MW=212.3, $CI/CH_4$ eE=70 eV) *m/z* 213 [$(M^+H)^+$, base peak], 193, 179, 167, 140, 139, 125, 98, 97.

**3-Thiophen-2-yl-2,2-dimethylpropionic acid (15)**

Ester **14** (16.4 g, 77.2 mmol) is hydrolyzed according to general procedure **G** to give **15** as a milky liquid (11.0 g, 77%). $^1H$ NMR ($CDCl_3$) 7.15 (d, 1; J=5.1), 6.95-6.90 (m, 1), 6.83 (d, 1, J=3.4), 3.10 (s, 2), 1.26 (s, 6); $^{13}C$ NMR

($CDCl_3$) 183.98, 139.37, 127.05, 126.66, 124.19, 43.55, 39.86, 24.69; MS (MW=184.3, EI, eE=70 eV) *m/z* 184 ($M^+$), 139, 123, 97 (base peak), 77, 69, 53, 45.

**2-(2-Isocyanato-2-methylpropyl)-thiophene (16)**

The carboxylic acid from the previous reaction (11.0 g, 60.0 mmol) is subjected to the Curtius rearrangement according to general procedure **H** to give the isocyanate **16** as a pale yellow liquid (9.94 g, 92%). $^1H$ NMR ($CDCl_3$) 7.22 (d, 1, J=5.1), 6.99, 6.89 (d, 1, J=3.5), 3.00 (s, 2), 1.38 (s, 6); $^{13}C$ NMR ($CDCl_3$) 138.20, 127.58, 126.74, 124.80, 58.12, 43.77, 29.92; IR ($CHCl_3$) 2982, 2259, 1265, 1167, 704; MS (MW=181.3, EI, eE=70 eV) *m/z* 181 ($M^+$), 149, 138, 127, 123, 99, 97 (base peak), 84, 77, 71, 58, 45.

**6,6-Dimethyl-6,7-dihydro-5H-thieno[3,2-c]pyridin-4-one (17)**

To a mixture of dry DCE (60 ml) and anhydrous $H_3PO_4$ (35 ml, prepared from 85% $H_3PO_4$ and $P_2O_5$) is added a solution of isocyanate **16** (5.12 g, 28.3 mmol) in DCE (20 ml). The resulting mixture is stirred vigorously at rt for 2 h, then at reflux for 4 h. The reaction mixture is allowed to cool and separate into two layers. The upper, organic layer is decanted, diluted with EtOAc and $Na_2CO_3$ solution, and extracted with EtOAc (2x). The organic extract is washed with brine (2x) and dried ($MgSO_4$), filtered, and evaporated. The residue is purified by FC (6:4 $CH_2Cl_2/CH_3CN$) to give a yellow solid, mp 153-154°C. Yield: 2.10 g (41%). $^1H$ NMR ($CDCl_3$) 7.43 (d, 1, J=5.2), 7.10 (d, 1, J=5.2), 6.82 (s, 1), 2.99 (s, 2), 1.38 (s, 6); $^{13}C$ NMR ($CDCl_3$) 162.64, 144.99, 130.86, 125.70, 122.96, 54.00, 37.31, 29.05; MS (EI, eE=70 eV) *m/z* 181 ($M^+$), 166, 151, 148, 125, 124 (base peak), 96, 83, 70, 45;

| | | | |
|---|---|---|---|
| Anal. Calcd for $C_9H_{11}NOS$ (MW=181.3) | C, 59.64 | H, 6.12 | N, 7.73. |
| Found | C, 59.76 | H, 6.17 | N, 7.87. |

**6,6-Dimethyl-4,5,6,7-tetrahydrothieno[3,2-c]pyridine**

Lactam **17** (2.76 g, 15.2 mmol) is reduced according to general procedure **I** to give a dark liquid (MW=167.3, 1.82 g, 71%). $^1H$ NMR ($CDCl_3$) 7.07 (d, 1, J=5.1), 6.75 (d, 1, J=5.1), 3.93 (s, 2), 2.66 (s, 2), 1.64 (br s, 1), 1.21 (s, 6).

**6,6-Dimethyl-6,7-dihydrothieno[3,2-c]pyridine N-oxide (18)**

The amine from the previous reaction (1.82 g, 10.9 mmol) is oxidized according to general procedure **E** to give the nitrone **18** as a yellow solid (660 mg, 33%, mp 130-131°C) after recrystallization from 4:1 hexane/$CH_2Cl_2$. $^1H$ NMR ($CDCl_3$) 7.72 (s, 1), 7.17 (d, 1, J=5.1), 6.89 (d, 1, J=5.1), 3.15 (s, 2), 1.50 (s, 6); $^{13}C$ NMR ($CDCl_3$) 131.24, 130.31, 128.63, 124.73, 123.54, 67.87, 37.41, 24.99; MS (EI, eE=70 eV) *m/z* 181 ($M_+$, base peak), 166, 149, 138, 134, 110, 96, 91, 77, 65, 51, 45;

| | | | |
|---|---|---|---|
| Anal. Calcd for $C_9H_{11}NOS$ (MW=181.3) | C, 59.64 | H, 6.12 | N, 7.73. |
| Found | C, 59.45 | H, 6.22 | N, 7.67. |

**REFERENCE EXAMPLE 5**

**5,5-Dimethyl-4,5-dihydrofuro[2,3-c]pyridine N-oxide (23)**

**3-Furan-3-yl-2,2-dimethylpropionic acid ethyl ester (19)**

Ethyl isobutyrate (10.7 ml, 80.1 mmol is alkylated with 3-(chloromethyl)furan according to general procedure **F** to give the product as a yellow liquid (12.91 g, 82%) after FC ($CH_2Cl_2$). $^1H$ NMR ($CDCl_3$) 7.32 (s, 1), 7.20 (s, 1), 6.20 (s, 1), 4.12 (t, 2, J=7.4), 2.66 (s, 2), 1.25 (t, 3, J=7.4), 1.18 (s, 6); $^{13}C$ NMR ($CDCl_3$) 177.56, 142.36, 140.54, 120.78, 112.76, 60.39, 42.85, 35.43, 24.96, 14.13; MS (MW=196.3, CI/$CH_4$, eE=70 eV) *m/z* 197 ($M_+H$)$_+$, 195, 161, 151, 123 (base peak), 109, 81.

**3-Furan-3-yl-2,2-dimethylpropionic acid (20)**

The ester from the previous reaction (12.9 g, 65.8 mmol) is hydrolyzed according to general procedure **G** to give a yellow liquid (10.24 g, 93%) after FC ($CH_2Cl_2$). $^1H$ NMR ($CDCl_3$) 7.34 (s, 1), 7.24 (s, 1), 6.25 (s, 1), 2.69 (s, 2),

1.22 (s, 6); $^{13}C$ NMR ($CDCl_3$) 184.25, 142.38, 140.60, 120.34, 112.14, 42.99, 35.27, 24.81; MS (MW=168.2, EI, eE=70 eV) *m/z* 168 ($M^+$), 123, 81 (base peak), 53.

**3-(2-Isocyanato-2-methylpropyl)-furan (21)**

The carboxylic acid from the previous reaction (10.2 g, 60.7 mmol) is subjected to the Curtius rearrangement according to general procedure **H** to give the isocyanate (8.56 g, 85%) as a yellow liquid. $^1H$ NMR ($CDCl_3$) 7.39 (s, 1), 7.31 (s, 1), 6.34 (s, 1), 2.63 (s, 2), 1.34 (s, 6); $^{13}C$ NMR ($CDCl_3$) 142.66, 140.96, 128.32, 119.64, 58.00, 39.35, 29.99; IR (film) 2962, 2930, 2257, 2172, 2135, 1717, 1489, 1208, 1186, 1163, 963; MS (MW=165.2, EI, eE=70 Ev) *m/z* 168 ($M^+$), 123, 81 (base peak), 53.

**5,5-Dimethyl-5,6-dihydro-4H-furo[2,3-c]pyridin-7-one (22)**

To a solution of $BF_3.Et_2O$ (2 ml, 160 mmol) in dry DCE (60 ml) at rt under $N_2$ is added a solution of isocyanate (6.60 g, 40.0 mmol) in DCE (20 ml) dropwise over 20 min. Stirring at rt is continued for 5 h. The reaction mixture is quenched by adding ice-cold $NaHCO_3$ solution. The mixture is diluted with EtOAc, washed with $NaHCO_3$ solution, dried ($MgSO_4$), filtered, and concentrated. The residue is crystallized from EtOAc to provide **22** (2.19 g, 33%) as a pale yellow solid, mp 133-134°C. $^1H$ NMR ($CDCl_3$) 7.53 (d, 1, J=1.8), 6.37 (d, 1, J=1.8), 5.50 (br s, 1), 2.77 (s, 2), 1.38 (s, 6); $^{13}C$ NMR ($CDCl_3$) 159.15, 146.02, 128.42, 111.71, 110.72, 54.83, 35.07, 29.35; MS (EI, eE=70 eV) *m/z* 165 ($M^+$), 150 (base peak), 132, 122, 108, 94, 80, 52, 42;

| | | | |
|---|---|---|---|
| Anal. Calcd for $C_9H_{11}NO_2$ (MW=165.2) | C, 65.44 | H, 6.71 | N, 8.48. |
| Found | C, 65.33 | H, 6.81 | N, 8.42. |

**5,5-Dimethyl-4,5,6,7-tetrahydrofuro[2,3-c]pyridine**

The lactam from the previous reaction (5.60 g, 3.39 mmol) is reduced according to general procedure **I** to give a dark liquid (MW=151.3, 2.46 g, 48%) which is not characterized, but used directly in the next step.

**5,5-Dimethyl-4,5-dihydrofuro[2,3-c]pyridine N-oxide (23)**

The amine from the previous reaction (2.42 g, 16.0 mmol) is oxidized according to general procedure **E**. The nitrone **23** is obtained as a yellow solid (1.35 g, 51%, mp 89-90°C) after FC (3:2 $CH_2Cl_2/CH_3CN$). $^1H$ NMR ($CDCl_3$) 7.68 (s, 1), 7.42 (s, 1), 6.40 (s, 1), 2.89 (s, 2), 1.48 (s, 6); $^{13}C$ NMR ($CDCl_3$) 145.01, 143.97, 124.05, 115.30, 110.91, 69.52, 34.67, 29.68, 25.45; MS (EI, eE=70 eV) *m/z* 165 ($M^+$, base peak), 150, 148, 133, 122, 105, 95, 91, 79, 77, 66, 65, 53, 51, 41;

| | | | |
|---|---|---|---|
| Anal. Calcd for $C_3H_{11}NO_2$ (MW=165.2) | C, 65.44 | H, 6.71 | N, 8.48. |
| Found | C, 65.43 | H, 6.70 | N, 8.51 |

## Claims

1. A compound selected from:

   2,2-dimethyl-1,2-dihydrobenzo[f]isoquinoline N-oxide **and**
   3,3-dimethyl-3,4-dihydrobenzo[h]isoquinoline N-oxide.

2. A pharmaceutical composition comprising a compound according to claim 1 in admixture with a pharmaceutically acceptable carrier.

3. A compound according to claim 1 for use as a medicament.

4. Use of a compound according to claim 1 in the manufacture of a medicament for the treatment of stroke.

5. Use of a compound according to claim 1 in the manufacture of a medicament for the treatment of myocardial infarction.

6. Use of a compound according to claim 1 in the manufacture of a medicament for the treatment of neurodegenerative disease.

7. Use of a compound according to claim 1 in the manufacture of a medicament for the treatment of septic shock.

8. Use of a compound according to claim 1 in the manufacture of a medicament for the treatment of tissue damage associated with physical trauma involving excessive bleeding.

9. Use of a compound according to claim 1 in the manufacture of a medicament for the treatment of atherosclerosis.

10. Use of a compound according to claim 1 in the manufacture of a medicament for use in inhibiting oxidative tissue damage.

**Patentansprüche**

1. Verbindung, ausgewählt aus:

   2,2-Dimethyl-1,2-dihydrobenzo[f]isochinolin-N-oxid und
   3,3-Dimethyl-3,4-dihydrobenzo[h]isochinolin-N-oxid.

2. Pharmazeutische Zusammensetzung, umfassend eine Verbindung nach Anspruch 1 in Beimengung zu einem pharmazeutisch verträglichen Träger.

3. Verbindung nach Anspruch 1 zur Verwendung als Arzneimittel.

4. Verwendung einer Verbindung nach Anspruch 1 bei der Herstellung eines Arzneimittels zur Behandlung von Schlaganfall.

5. Verwendung einer Verbindung nach Anspruch 1 bei der Herstellung eines Arzneimittels zur Behandlung von Herzinfarkt.

6. Verwendung einer Verbindung nach Anspruch 1 bei der Herstellung eines Arzneimittels zur Behandlung von neurodegenerativer Erkrankung.

7. Verwendung einer Verbindung nach Anspruch 1 bei der Herstellung eines Arzneimittels zur Behandlung von septischem Schock.

8. Verwendung einer Verbindung nach Anspruch 1 bei der Herstellung eines Arzneimittels zur Behandlung von physischem Trauma zugehöriger Gewebsschädigung, die starke Blutungen einbezieht.

9. Verwendung einer Verbindung nach Anspruch 1 bei der Herstellung eines Arzneimittels zur Behandlung von Atherosklerose.

10. Verwendung einer Verbindung nach Anspruch 1 bei der Herstellung eines Arzneimittels zur Verwendung bei der Hemmung oxidativer Gewebsschädigung.

**Revendications**

1. Composé choisi parmi :

   le 2,2-diméthyl-1,2-dihydrobenzo[f]isoquinoléine-N-oxyde et
   le 3,3-diméthyl-3,4-dihydrobenzo[h]isoquinoléine-N-oxyde.

2. Composition pharmaceutique comprenant un composé selon la revendication 1 en mélange avec un support pharmaceutiquement acceptable.

3. Composé selon la revendication 1, destiné à être utilisé en tant que médicament.

4. Utilisation d'un composé selon la revendication 1 pour la fabrication d'un médicament destiné au traitement de l'attaque cérébrale.

5. Utilisation d'un composé selon la revendication 1 pour la fabrication d'un médicament destiné au traitement de l'infarctus du myocarde.

6. Utilisation d'un composé selon la revendication 1 pour la fabrication d'un médicament destiné au traitement d'une maladie neurodégénérative.

7. Utilisation d'un composé selon la revendication 1 pour la fabrication d'un médicament destiné au traitement du choc septique.

8. Utilisation d'un composé selon la revendication 1 pour la fabrication d'un médicament destiné au traitement d'une lésion tissulaire associée à un traumatisme physique impliquant un saignement excessif.

9. Utilisation d'un composé selon la revendication 1 pour la fabrication d'un médicament destiné au traitement de l'athérosclérose.

10. Utilisation d'un composé selon la revendication 1 pour la fabrication d'un médicament destiné à être utilisé pour l'inhibition d'une lésion tissulaire oxydative.